# EUROPEAN PATENT SPECIFICATION

(11) **EP 3 775 903 B1**
(45) Date of publication and mention of the grant of the patent: **02.04.2025**
(21) Application number: 19716211.8
(22) Date of filing: 12.04.2019
(51) Int. Cl.: G01N 33/569, G01N 33/68

(54) **MAMMALIAN MHC PEPTIDE DISPLAY AS AN EPITOPE SELECTION TOOL FOR VACCINE DESIGN**
SÄUGETIER-MHC-PEPTID-ANZEIGE ALS EPITOP-AUSWAHLWERKZEUG FÜR IMPFSTOFFDESIGN
AFFICHAGE DE PEPTIDES MHC DE MAMMIFÈRE TEL QU'UN OUTIL DE SÉLECTION D'ÉPITOPE POUR LA CONCEPTION DE VACCINS

(30) Priority: 12.04.2018 EP 18167050
(43) Date of publication of application: 17.02.2021
(73) Proprietor: ETH Zurich, 8092 Zürich (CH)
(72) Inventor: KISIELOW, Jan, 8952 Schlieren (CH); OBERMAIR, Franz Josef, Oberengstringen 8102 (CH); KOPF, Manfred, 8006 Zürich (CH)
(74) Representative: Vossius & Partner Patentanwälte Rechtsanwälte mbB
(86) International application number: PCT/EP2019/059563
(87) International publication number: WO 2019/197671

(56) References cited:
- WO-A1-2016/097334
- WO-A2-2004/015395
- US-A1- 2006 099 218
- FEI WEN ET AL: "Cell surface display of functional human MHC class II proteins: yeast display versus insect cell display", PROTEIN ENGINEERING, DESIGN AND SELECTION, vol. 24, no. 9, 13 July 2011 (2011-07-13), GB, pages 701 - 709, XP055608563, ISSN: 1741-0126, DOI: 10.1093/protein/gzr035
- CARSTEN LINNEMANN ET AL: "TCR repertoires of intratumoral T-cell subsets", IMMUNOLOGICAL REVIEWS., vol. 257, no. 1, 13 January 2014 (2014-01-13), US, pages 72 - 82, XP055316446, ISSN: 0105-2896, DOI: 10.1111/imr.12140

## Description

The present invention relates to a method for identifying candidate peptides presented by the major histocompatibility complex (MHC) for *in vivo* and/or *in vitro* interventions including vaccination, induction of immunological tolerance, blocking of TCRs and MHC-mediated toxin delivery, for immunogenicity testing and other *in vitro* T-cell reactivity tests.

T-cells play a central role in the immune system. The functions of T-cells depend critically on the recognition of epitopes contained in peptides presented by antigen presenting cells (APCs). APCs can be almost any nucleated cell type, but professional APCs such as dendritic cells (DCs) are particularly efficient in presentation. There are different sets of T-cells with different functions, in particular CD4+ T-cells or CD8+ T-cells which recognize MHCII-bound epitopes presented by professional APCs or MHCI-bound epitopes presented by most cell types, respectively. MHC molecules play an important role in presenting cellular antigens or epitopes in the form of short linear peptides to T cells.

The MHC consists of alpha and beta chains, and a peptide bound in a groove formed by these chains. The stability of this complex is highly dependent on peptide binding, so that empty MHC molecules are downregulated from the cell surface and degraded. Nevertheless, empty MHC molecules exist on the surface of cells and can bind extracellular peptides and present them to T cells. Furthermore, two distinct isomers of empty MHC were described (Rabunowitz et al., Immunity, 9, 699-709 (1998)): a less stable *active* isomer and a more stable *inactive* isomer. An individual's MHC repertoire is polygenic and MHC genes are highly polymorphic. The set of MHCs an individual can express is therefore most likely very different from the set of another random individual.

Peptide carrying MHCs interact with T cell receptors (TCRs) present on the surface of T cells, which leads to T cell activation. APCs such as dendritic cells or macrophages, can internalize antigens by phagocytosis or by receptor-mediated endocytosis.

To control the activity of T-cells, it is important to determine their specific peptide-ligands and control or modulate the interaction of the MHC-peptide complexes with the TCRs. It is therefore important to determine the peptide binding affinity to an MHC. Suitable peptides or MHC-peptide complexes may be used for a variety of applications. They may be used, for example, to identify or enrich reactive T-cells or for immunogenicity testing *in vitro.* They may be also used to block TCRs, to deliver toxins to T-cells *in vivo* or to redirect T cells with MHC-containing chimeric antigen receptors, the MHC-CARs (Jyothi et al., Nat.Biotech, 20, 1215-1220 (2002)), to induce immunological tolerance to an epitope which otherwise would activate T-cells specific for this epitope. The development of vaccines, in particular tumor-specific antigen (TSA)-based cancer vaccines to activate T-cells for the elimination of cancer cells, is among the major goals of modern medicine. Tumor-specific peptides, so-called neoantigens, are only present in tumor cells and entirely absent from the normal tissue. Such tumor-specific peptides are created by tumor-specific DNA alterations that result in the formation of novel protein sequences. Due to the genetic heterogeneity of cells within and between different tumors, even when they originate form the same organ, the set of neoantigens present in a tumor of a patient is largely specific to this patient. Tumor-specific peptides are displayed in the context of the MHC on the surface of tumor cells and so-called antigen presenting cells (APCs). Once tumor-specific peptides are presented on the cell surface they can be recognized and destroyed by the immune system. Thus, vaccines containing tumor-specific peptides can stimulate the immune system to detect and destroy cancer cells that present these molecules on their surface.

WO 2016/097334 A1 discloses a chimeric antigen-receptor polypeptide heterodimer comprising two polypeptides, wherein the first contains an extracellular part of the major histocompatibility complex I alpha chain and the second contains amicroglobulin domain, or the first contains an extracellular part of the major histocompatibility complex II alpha chain and the second contains a major histocompatibility complex II beta chain.

WO 2004/015395 A2 is directed to a method for identifying MHC-presented peptide epitopes for T cells in insect cells.

US 2006/099218 A1 discloses methods for enhancing exogenous epitope display on an MHC class I molecule through inhibition of TAP activity.

Wen et al., (PROTEIN ENGINEERING, DESIGN AND SELECTION, GB, (20110713), vol. 24, no. 9, doi:10.1093/protein/gzr035, ISSN 1741-0126) discloses cell surface display of functional human MHC class II proteins in yeast and insect cells.

Linnemann et al., (IMMUNOLOGICAL REVIEWS., US, (20140113), vol. 257, no. 1, doi:10.1111/imr.12140, ISSN 0105-2896) discloses TCR repertoires of intratumoral T-cell subsets.

Recently it has been shown that TSAs arising from somatic mutations accumulating in tumors are frequently recognized by tumor-infiltrating lymphocytes (TILs) (Linnemann et al., Nat Med 1-7 (2014)). Exome sequencing of the tumor DNA and comparison with DNA from healthy tissue allows the discovery of such somatic mutations specific to the tumor cells of a patient. However, usually many mutations are detected (Vormehr et al., Curr Opin Immunol 39, 14-22 (2016)) but only few TSAs (i.e. tumor specific peptides) should be mixed in one vaccine, due to technical issues, but also to minimize the risk of autoimmunity. The selection of these TSAs is crucial because antigen-presenting cells (APCs) do not present all possible peptides on their MHC molecules, but rather a subset that fits the particular MHC allele, which is specific to the patient. Choosing peptides, in particular tumor specific peptides, that would be efficiently presented by APCs is critical for many applications involving T-cells and remains a bottleneck in tumor vaccine design. To solve this problem, candidate peptides are usually ranked according to their MHC binding affinity predicted with the help of bio-informatic analysis (Sahin et al., Nature 1-19 (2017); Ott et al., Nature 547, 217-221 (2017)). However, recent data indicates that bio-informatic prediction of peptide presentation performs rather poorly, in particular for MHC class II epitopes (Sofron et al., Eur J Immunol 46, 319-328 (2015)). A "wet-lab" method using an antigen-presenting reporter cell line to measure the binding of a T cell to the MHC-peptide complex (via its TCR) has been disclosed; WO2016097334 A1. That reporter cell line expresses a chimeric antigen receptor comprising a MHC-peptide complex which activates a reporter gene in the reporter cell line upon binding to the TCR of a T cell. Indirectly, also the stability of the MHC-peptide complex is measured with that method, because only sufficiently stable MHC-peptide complexes will emerge at the surface of the reporter cell line, interact with T cells and eventually activate the reporter. However, that method does not allow specifically measuring the MHC-peptide complex stability, because activation of the reporter gene also depends on the affinity of the TCRs to the epitopes of the peptide and other immune-modulating factors.

There is therefore a need for providing means and methods for reliably determining peptides, in particular TSAs, that are efficiently presented by APCs.

The present invention now satisfies this need in that it provides such means and methods, which are more specifically defined in the claims and the following embodiments of the invention.

In its main aspects the invention relates to:
1. A method for identifying a candidate peptide to be presented by a major histocompatibility complex (MHC), the method comprising the steps of:
   a. expressing a recombinant MHC-peptide complex in a reporter cell line, wherein the recombinant MHC-peptide complex is a peptide-MHC-TCR chimera in which the candidate peptide is covalently bound to the MHC and wherein the reporter cell line is a mammalian cell line;
   b. detecting and isolating reporter cells that express one or more recombinant MHC-peptide complex(es) on the cell surface, wherein the detection and isolation of reporter cells comprises a step of contacting the reporter cells with an antibody directed against the recombinant MHC-peptide complex;
   c. determining the amino acid sequence of the candidate peptide presented on the cell surface of a reporter cell isolated in step (b); and
   d. identifying the candidate peptide of step (c) to be presented by an MHC.
2. The method according to aspect 1, wherein the MHC is:
   a. an MHC class II molecule comprising an extracellular MHC class II alpha chain and a transmembrane domain, and an extracellular MHC class II beta chain and a transmembrane domain; or
   b. an MHC class I molecule comprising an extracellular MHC class I alpha chain and a transmembrane domain, and a beta-2 microglobulin.
3. The method according to aspect 2, wherein the MHC class I alpha chain comprises a Y84A mutation.
4. The method according to aspects 2 or 3, wherein the transmembrane domain is a native transmembrane domain of an MHC molecule or a transmembrane domain of a heterologous molecule, in particular wherein the heterologous molecule is TCRα/β, TCRγ/δ, CD3γ/δ/ε/ζ, CD4 or CD8α/β.
5. The method according to any one of aspects 1 to 4, wherein the antibody is directed against MHC-I or MHC-II, or wherein the antibody is directed against CD3γ, CD3δ or CD3ε.
6. The method according to any one of aspects 1 to 5, wherein reporter cells efficiently expressing a recombinant MHC-peptide complex on their cell surface are isolated by FACS or MACS.
7. The method according to any one of aspects 1 to 6, wherein the reporter cell line is a cell line lacking the MHC class II peptide loading machinery and/or lacking a functional TAP1 , TAP2 and/or beta-2-microglobulin gene.
8. The method according to any one of aspects 1 to 7, wherein the reporter cell line is a T cell hybridoma cell line.
9. The method according to any one of aspects 1 to 8, wherein the candidate peptide is encoded in an MHC-peptide complex library.
10. The method according to aspect 9, wherein the MHC-peptide library is obtained by cloning a plurality of polynucleotides encoding the candidate peptides upstream of and in-frame with a recombinant MHC beta and/or alpha chain.
11. The method according to aspect 10, wherein the plurality of polynucleotides encoding the candidate peptides is obtained:
   a. by mutagenesis of one or more polynucleotides encoding native peptide(s); or
   b. by random sheering or digestion of cDNA or DNA derived from cells or pathogens of interest.
12. The method according to any one of aspects 9 to 11, wherein the MHC-peptide library encodes at least 10, at least 50, at least 200 or at least 1000 peptide candidates.
13. The method according to any one of aspects 9 to 12, wherein the MHC-peptide library is encoded by a plurality of viral vectors.
14. The method according to any one of aspects 1 to 13, the method comprising an additional step of determining the level of cell surface expression of the recombinant MHC-peptide complex.
15. The method according to any one of aspects 1 to 14, wherein the candidate peptide is a tumor-specific peptide carrying individual tumor-derived mutation(s).

The present invention provides methods for identifying candidate peptides presented by major histocompatibility complex (MHC) comprising expressing in a reporter cell line a recombinant MHC-peptide complex comprising a covalently bound candidate peptide, detecting reporter cells that show surface expression of the MHC-peptide complex, and determining the sequence of candidate peptides presented at the cell surface.

The invention is, at least partly, based on the surprising discovery that the surface expression level of a recombinant peptide-MHC complex in a mammalian cell line can be directly measured in a quantitative way. It is further surprising that such a direct measurement of a peptide-MHC complex was feasible by a standard laboratory technique based on antibody staining and flow cytometry, and without a complex co-culture system involving T cells and/or a sophisticated reporter system. Although it may be known to a person skilled in the art that predictions based on bioinformatics approaches are not always very accurate, it is still surprising that a state-of-the art bioinformatics method completely failed to predict the binding of a peptide to specific MHC variants as determined by the method disclosed herein. Further surprising is that the detection of a MHC comprised in a recombinant MCR (peptide-MHC-TCR chimera disclosed in WO2016097334 A1) complex was possible with a CD3e antibody regardless of the specific peptide and/or MHC variant to be analyzed. Without being bound by theory, the method disclosed herein allows to measure primarily the biological variability of peptide presentation by MHCs without the technical variability which may be introduced when different antibodies are used to detect different MHC variants.

Peptides can be covalently attached to MHC molecules to overcome the need of peptide processing/loading machinery. This approach has been used to produce stable MHC-tetramers (Crawford et al., Immunity 8, 675-682 (1998)), in various MHC-display methods (Crawford et al., PLoS Biol 2, e90 (2004); Wen et al., Protein Eng Des Sel 24, 701-709 (2011)), to generate mice expressing single peptides on their APCs (Ignatowicz et al.,Cell 84, 521-529 (1996)) or in CARs designed to redirect T-cells towards other T cells (Jyothi et al., Nat Biotechnol 20, 1215-1220 (2002)). Often, MHC molecules had to be mutated to allow expression on the surface of yeast or insect cells (Wen et al., Protein Eng Des Sel 24, 701-709 (2011)) and in some studies measures were taken to stabilize the peptides in the groove of the MHC by incorporating an additional disulfide-bridge (Truscott et al., J. Immunol. 178, 6280-6289 (2007)).

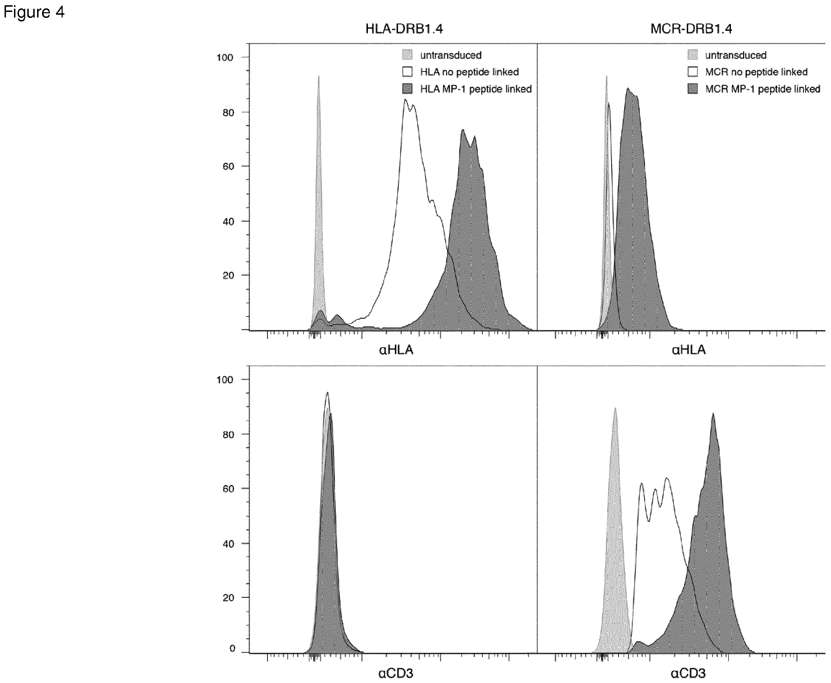

However, it is shown within the context of the present invention that, in cells incapable of MHC peptide loading, recombinant MHC molecules without tethered peptides, are undetectable or unstable (detectable at low levels) on the cell surface, even if over-expressed (Figure 4).

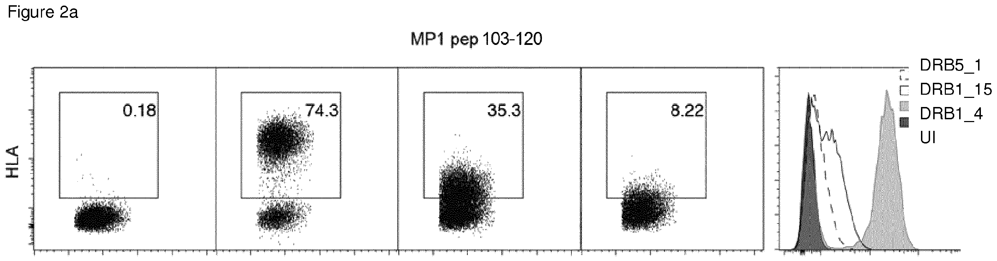

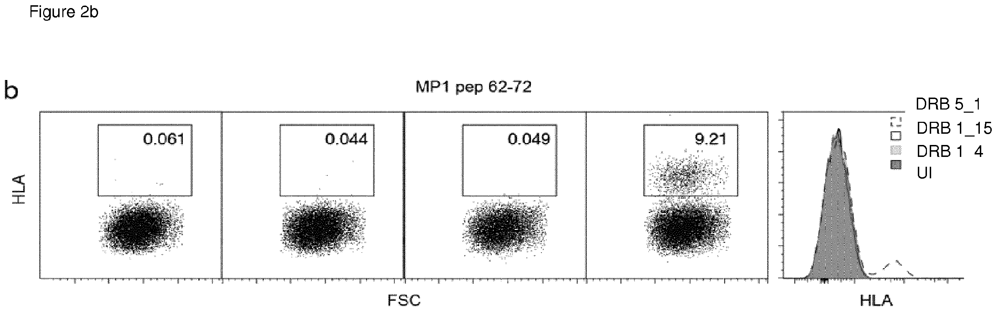

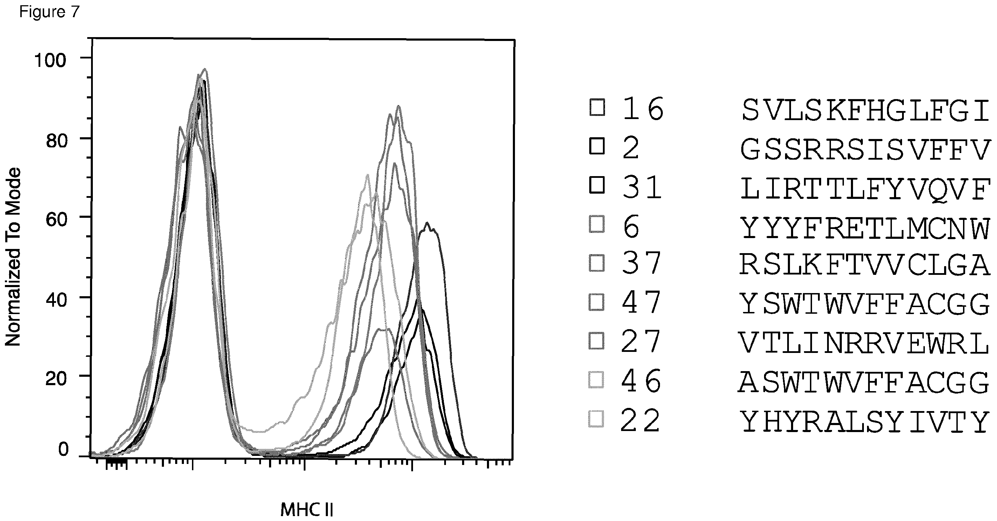

So, the relatively stable isomers of empty MHC described by Rabunowitz (Rabunowitz et al., Immunity, 9, 699-709 (1998)), even if present, do not persist very long on the surface of non-professional antigen presenting cells. Importantly, it is shown within the context of the present invention that not all peptides tethered to the recombinant MHC lead to efficient cell surface expression of the MHC complex. In fact, some prevent expression on the surface (Figures 2a and b). Thus, high expression of recombinant MHC molecules with tethered candidate peptides on the surface of appropriate reporter cell lines compared to uninfected reporter cells and/or reporter cells transduced with MHC without covalently bound peptide or unstable MHC-peptide complexes can be used as an indicator of efficient peptide presentation by the MHC. In other words, reporter cells showing surface expression of a recombinant MHC-peptide complex can be detected by comparing the surface expression of said MHC-peptide complex to the background signal of uninfected reporter cells, or reporter cells transduced with an MHC without covalently bound peptide or an unstable MHC-peptide complex. Furthermore, measurement of the level of such presentation/expression (Figures 2a and 7) allows the formation of a ranking of peptides according to their MHC binding abilities. The detection and/or ranking of peptides presented by MHCs may comprise, whenever necessary, one or more normalization steps, for example normalization by the background signal of the respective MHC variant having no candidate peptide covalently bound or exerting an unstable interaction with a candidate peptide or a random peptide. Normalization and/or standardization methods, which are well known in the art, can be used, whenever necessary, to determine the binding affinity of a candidate peptide to an MHC based on the measured surface expression of the recombinant MHC-peptide complex.

In certain embodiments, the ranking of candidate peptides and/or the determination of the binding affinity of a candidate peptide to an MHC can be directly correlated with the surface expression of a recombinant MHC-peptide complex determined by CD3 staining using an anti-CD3e, anti-CD3δ or anti-CD3γ antibody. Preferably, the MHC is comprised in an MCR for said correlation with said CD3 staining.

It was now shown for the first time that a properly designed MHC class II-peptide complex can be used to identify candidate peptides efficiently presented by APCs.

Furthermore, it was found within the scope of the present invention that not all MHC-class II peptide combinations are efficiently expressed on the surface of cells, when MHC-TCR fusion proteins (MCRs) are used, which are composed of peptides linked to native extracellular domains of the MHC fused to trans-membrane units of the TCR. For example, differential surface expression of the influenza matrix protein 1 (MP1)-derived peptide was observed when different MHC-peptide combinations were tested. Importantly, peptide-binding prediction analyses did not indicate a similar pattern for MHC peptide binding affinity.

In various embodiments, the invention relates to a method for identifying candidate peptides according to the preceding embodiment as described herein, wherein the method comprises the following steps:
(i) generating libraries comprising candidate peptides, particularly libraries comprising candidate peptides cloned upstream of and in-frame with a recombinant MHC beta and/or alpha chain;
(ii) transducing such libraries, together with the corresponding MHC alpha and/or beta chain, into suitable reporter cell lines;
(iii) detecting cells that present one or more MHC-peptide complex(es) on their cell surface;
(iv) isolating DNA from cells that present one or more MHC-peptide complex(es) on their cell surface;
(v) determining the sequence of the candidate peptide within the MHC-peptide complex presented on the cell surface.

As used herein, the term "major histocompatibility complex (MHC)" refers to the MHC Class I and MHC Class II genes and the proteins encoded thereby. The terms MHC-I, MHC-II, MHC-1 and MHC-2 are variously used herein to indicate these classes of molecules.

In certain embodiments, the invention relates to a method for identifying candidate peptides presented by major histocompatibility complex (MHC) comprising expressing in a reporter cell line a recombinant MHC-peptide complex comprising a covalently bound candidate peptide, detecting reporter cells that show surface expression of the MHC-peptide complex, and determining the level of the surface expression of the MHC-peptide complex, and the sequence of candidate peptides presented at the cell surface.

In various embodiments, the invention relates to a method for identifying candidate peptides according to the preceding embodiment as described herein, wherein the method comprises the following steps:
(i) generating libraries comprising candidate peptides cloned upstream of and in-frame with a recombinant MHC beta and/or alpha chain;
(ii) transducing such libraries, together with the corresponding MHC alpha and/or beta chain, into suitable reporter cell lines;
(iii) detecting and isolating cells that express one or more MHC-peptide complex(es) on the cell surface;
(iv) determining the level of cell surface expression of the one or more MHC-peptide complex(es)
(v) isolating DNA from cells that present one or more MHC-peptide complex(es) on their cell surface;
(vi) determining the sequence of candidate peptides encoded by the vectors integrated in the DNA isolated from cells that present MHC-peptide complex(es) on the cell surface.

In mammals, there are two types of MHC molecules designated class I and class II. MHC class I molecules are present on almost all nucleated cells in the body and are recognized by CD8+ cells. MHC class II molecules are mainly expressed on professional APCs of the immune system and are recognized by CD4+ cells. The molecules are further subdivided by antigenic subtype. Human MHC class I molecules, also referred to as human leukocyte antigens (HLA), are designated HLA-A, -B, and -C. Human MHC class II molecules are designated HLA-DR, -DQ, and - DP.

MHC class I molecules are found on almost every cell of the body. MHC class I molecules are heterodimers that are composed of a polymorphic heavy chain (α) non-covalently associated with a monomorphic (in humans) non-MHC encoded light (β) chain protein of about 12 kDa, termed β₂ microglobulin (β₂m). The heavy α chain is a polymorphic transmembrane glycoprotein of about 45kDa consisting of 3 extracellular domains, each containing about 90 amino acids (α₁ at the N-terminus, α₂ and α₃), a transmembrane region of about 40 amino acids and a cytoplasmic tail of about 30 amino acids. The α₁ and α₂ domains, the membrane distal domains, form the peptide-binding groove or cleft having a sufficient size to bind a peptide of 8-10 amino acids, whereas the α₃ domain is proximal to the plasma membrane. The peptide being presented is held by the peptide-binding groove, in the central region of the α₁ and α₂ domains.

MHC Class II molecules are found only on a few specialized cell types, particularly antigen-presenting cells (APCs) such as macrophages, dendritic cells, and B cells. Class II MHC molecules contain two different polypeptide chains, a 33-kD α chain and a 28-kDa β chain, which associate by non-covalent interactions. Like class I MHC molecules, class II MHC molecules are membrane-bound glycoproteins that contain extracellular domains, a transmembrane segment and a cytoplasmic tail.

Each chain in these non-covalent heterodimeric complexes contains two extracellular domains, a transmembrane domain and a cytoplasmic tail. The extracellular domains are α₁ and α₂ domains and β₁ and β₂ domains for the α chain and β chain, respectively. The membrane-distal domain of a class II molecule is composed of the α₁ and β₁ domains and forms the peptide-binding groove or cleft having a sufficient size to bind a peptide, which is typically 13-18 amino acids in length, alternatively 10-18 amino acids or longer. The membrane-proximal pairs of class **II,** α₂ and β₂, have structural similarities to Ig constant (C) domains. Three pairs of class II α and β chain genes exist in humans, known as HLA-DR, HLA-DP and HLA-DQ. The highest level of polymorphism is documented for HLA-DR.

As used herein, a candidate peptide refers to a peptide with 8-18 amino acids or up to 50 amino acids in length. The preferred length of a candidate peptide binding to MHC class I is 8, 9 or 10 amino acids. The preferred length of a candidate peptide binding to MHC class II is 10, 11, 12, 13, 14, 15, 16, 17 or 18 amino acids. In particular, a candidate peptide is comprised in a MHC-peptide complex encoded by a recombinant nucleic acid.

In various embodiments, the invention relates to a method according to any one of the preceding embodiments as described herein, wherein the MHC molecule is a MHC class II molecule comprising the extracellular MHC class II alpha chain and a transmembrane domain, as well as the extracellular MHC class II beta chain and a transmembrane domain.

In other embodiments, the MHC molecule is a MHC class I molecule, comprising the extracellular MHC class I alpha chain and a transmembrane domain, as well as the beta-2 microglobulin.

In yet another embodiment, the MHC-peptide complex is a fusion protein comprising the candidate peptide, beta-2 macroglobulin, the extracellular MHC class I alpha chain and a transmembrane domain.

For MHC class I molecules it has been shown that a Y84A mutation in the alpha chain opens the peptide binding pocket enabling better accommodation of the linked peptide (Mitaksov, V. et al. Chem Biol 14, 909-922 (2007)).

Accordingly, in a specific embodiment, the MHC alpha chain of the MHC class I molecule carries the Y84A mutation. For example, the Y84A mutation can be introduced by site-directed mutagenesis.

In some embodiments, the invention relates to a method according to any one of the preceding embodiments as described herein, wherein the MHC molecule comprises one or two transmembrane domains.

In various embodiments, the invention relates to a method according to any one of the preceding embodiments as described herein, wherein the transmembrane domain is the native transmembrane domain of the MHC molecule.

As used herein, the term "native transmembrane domain" refers to a MHC transmembrane domain that maintains the structural property of the transmembrane portion of a MHC molecule in its native state.

In specific embodiments, the transmembrane domain is a transmembrane domain of a heterologous molecule including but not limited to TCRα/β, TCRγ/δ, CD3γ/δ/ε/ζ, CD4 or CD8α/β.

The reporter cell line of the present invention is a mammalian cell line.

Suitable mammalian cell lines are for example, but not limited, to T cell hybridomas, T cell clones, Jurkat, BW5147α-β-fusion partner, myelomas, HEK, CHO, 3T3.

In particular, a mammalian reporter cell line suitable for use in the present invention is one lacking the ability to display endogenous protein-derived peptides originally existing in the cell. Thus, the cell should only present MHC-peptide complexes that carry candidate peptides derived from the peptide library of interest. The processing of peptides and their loading on MHC molecules for display is a multistep process involving multiple molecular species that constitute the processing and presenting machinery. MHC class I molecules are loaded with peptides via a transporter called TAP (transporter associated with antigen processing) which translocates peptides into the ER, where they bind the MHC class I molecules. Loading of a MHC class II molecule occurs by phagocytosis or autophagy; MHC class II molecules are delivered to the phagolysosomes and loaded with peptides prior to their migration to the cell surface. The peptide processing/loading machinery for MHC class II molecules is only present in APCs.

In accordance with the above, in one embodiment, a reporter cell line suitable for carrying out the method of the invention is one lacking the MHC class II peptide loading machinery. Preferably, the reporter cell line lacking the MHC class II peptide loading machinery is a mammalian cell line.

In a specific embodiment, the reporter cell line is a T-cell hybridoma.

In another embodiment, a reporter cell line suitable for carrying out the method of the invention is one lacking a functional TAP1, TAP2 and/or beta-2-microglobulin gene.

In a specific embodiment, the reporter cell line is a T-cell hybridoma with a defective or deleted TAP1, TAP2 and/or beta-2-microglobulin gene. Other examples of reporter cells lines with a defective or deleted TAP1, TAP2 and/or beta-2-microglobulin gene include but are not limited to T- and B-cell clones or hybridomas, HEK cells, or 3T3 cells.

Exome sequencing may be used to identify TSAs that are uniquely present in a tumor. For example, TSAs may be identified by sequencing the tumor DNA derived from patients and comparing it with DNA derived from healthy subjects. TSAs may also be identified for single patients by sequencing the tumor and normal DNA of each patient. Based on this analysis, a plurality of candidate peptides each carrying tumor-derived mutation(s) may be used for the generation of MHC-peptide complexes. Thus, in one embodiment of the invention, a library of peptides is used. These peptides may carry tumor derived mutation(s) or be native peptides.

Accordingly, in various embodiments, the invention relates to a method according to any one of the preceding embodiments as described herein, wherein the candidate peptide is a tumor-specific peptide carrying individual tumor-derived mutation(s).

The term "tumor-derived mutation" as used herein refers to mutations in nucleic acid sequences that are specific to a tumor and absent in DNA from healthy tissue.

In a specific embodiment, the tumor-derived mutation is a single nucleotide variant (SNV). SNV include but are not limited to various mutations of p53, KRAS, and BRAF.

In another embodiment, the candidate peptide is an antigen that causes an immune response. For example, the peptides can be derived from pathogens.

In yet another embodiment, the candidate peptide is a compound undergoing immunogenicity testing.

In one embodiment, the invention relates to a method using a library of candidate peptides, wherein the library comprises mutant forms of native peptide(s). For example, the native peptide(s) may be known to be not efficiently presented by a given MHC molecule. Thus, in order to screen for peptide mutants which are more efficiently presented by a given MHC molecule, a library comprising mutant peptides may be used.

In another embodiment, the invention relates to a method using a library of candidate peptides, wherein the library comprises peptides generated by random sheering or digestion of cDNA or DNA derived from cells or pathogens of interest. Such a library would cover all peptides present in such cells. For example, generating MHC display libraries from cancer tissue or tissue undergoing auto-immune attack, would allow the definition of all tissue peptides possibly presented by a given MHC. Thus, in future no tests would need to be performed and whether a particular peptide is efficiently presented by a given MHC could be looked up in a table.

In one embodiment, the MHC-peptide complex library is generated using recombinant expression vectors. Recombinant expression vectors are replicable DNA constructs comprising an assembly of (1) agent(s) having a regulatory role in gene expression, for example, promoters, operators, or enhancers, operatively linked to (2) a nucleotide sequence encoding a desired protein (such as the MHC-peptide complex) which is transcribed into mRNA and translated into protein, and (3) appropriate transcription and translation initiation and termination sequences. The choice of promoter and other regulatory elements generally varies according to the intended reporter cell line. Expression vectors are often in the form of "plasmids" which refer to circular double stranded DNA loops which, in their vector form are not bound to the chromosome. Suitable prokaryote expression vectors include plasmids from E. coli, e.g. Col E1, pCR1, pBR322, pMB9 and their derivatives, wider host range plasmids, e.g. RP4, phage DNAs, such as phage lambda and its various derivatives, M13, and the like. Additional E. coli vectors are described for example in Maniatis et al., Molecular Cloning: A Laboratory Manual, Cold Spring Harbor, NY (1982) and Sambrook et al., Molecular Cloning: A Laboratory Manual, Cold Spring Harbor, NY (1989). However, the invention is intended to include such other forms of expression vectors which serve equivalent functions and which become known in the art subsequently hereto. Eukaryote expression vectors, replicating episomally, such as pCEP4 or BKV, or other vectors derived from viruses, such as retroviruses e.g. pMY, pMX, pSIR, adenoviruses e.g pAd, and the like, may be employed. In the expression vectors, regulatory elements controlling transcription or translation can be generally derived from mammalian, microbial, viral or insect genes. The ability to replicate, usually conferred by an origin of replication, and a selection gene to facilitate recognition of transformants may additionally be incorporated. Expression vectors containing regulatory elements from eukaryotic viruses are typically used in eukaryotic expression vectors, e.g., SV40 vectors, papilloma virus vectors, and vectors derived from Epstein-Barr virus. Other exemplary eukaryotic vectors include pMSG, pAV009/A+, pMTO10/A+, pMAMneo-5, baculovirus pDSVE, and any other vector allowing expression of proteins under the direction of the CMV promoter, SV40 early promoter, SV40 later promoter, metallothionein promoter, murine mammary tumor virus promoter, Rous sarcoma virus promoter, polyhedrin promoter, or other promoters shown effective for expression in eukaryotic cells.

A "promoter" is defined as an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription. Promoters for use in eukaryotic host cells are known to those skilled in the art. Illustrative examples of such promoters include, but are not limited to, promoters from Simian Virus 40 (SV40), Mouse Mammary Tumor Virus (MMTV) promoter, Human Immunodeficiency Virus (HIV) promoters, such as the HIV Long Terminal Repeat (LTR) promoter, Moloney virus promoters, ALV promoters, cytomegalovirus (CMV) promoters, such as the CMV immediate early promoter, Epstein Barr Virus (EBV) promoter, Raus Sarcoma Virus (RSV) promoter, as well as promoters from human genes such as human actin, human myosin, human hemoglobin, human muscle creatine, and human metalothionein. Still other examples of suitable promoters include the CAG promoter (a hybrid promoter comprising a CMV enhancer, a chicken β-actin promoter, and a rabbit β-globin splicing acceptor, and poly(A) sequence).

The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

The MHC-peptide library is introduced into suitable reporter cell lines by transducing the expression vector into the host cell using standard techniques known in the art.

Suitable methods are, for example, described in Sambrook et al., Molecular Cloning: A Laboratory Manual, Cold Spring Harbor, NY (1989). To produce pseudo-retroviruses for transduction, packaging cell lines constantly expressing retroviral proteins GAG, POL and ENV (like for example the Phoenix cell line), are transiently transfected with constructs containing the viral genome composed of the LTRs, packaging signals and the genes of interest (in this case the peptide carrying MHC chains). Alternatively, suitable cell lines, like HEK, 3T3 or other, are transiently transfected with a mixture of vectors encoding separately the retroviral proteins GAG, POL and ENV and the viral genome composed of the LTRs, packaging signals and the genes of interest. These commonly used strategies ensure the production of defective pseudo-retroviruses which are able to infect target cells and introduce the genes of interest into their genomic DNA. However, the infected target cells are not able to produce retroviruses because the pseudo-retroviruses do not carry the gag, pol and env genes in their genome. Alternatively, the MHC-peptide libraries can be introduced into suitable reporter cell lines by transfection with reagents based on lipids, calcium phosphate, cataionic polymers or DEAE-dextran, or by electroporation.

The terms "infection" and "transduction" of a reporter cell line are used herein interchangeably.

Methods for detecting cells that present cell surface-exposed MHC-peptide complexes are known in the art. Suitable methods include cell sorting techniques, such as flow cytometry, fluorescence-activated cell sorting (FACS), and magnetic cell sorting (MACS).

Accordingly, in various embodiments, the invention relates to a method according to any one of the preceding embodiments as described herein, wherein reporter cells efficiently expressing MHC on their surface are enriched by fluorescence activated cell sorting (FACS).

FACS refers to a method of separating a population of cells into one or more sub-populations based on the presence, absence, or level of one or more specific polypeptides expressed by the cells. FACS relies on optical properties, including fluorescence, of individual cells in order to sort the cells into sub-populations. Cell sorters suitable for carrying out a method described herein are well-known in the art and commercially available. Exemplary cell sorters include MoFlo sorter (DakoCytomation, Fori Collins, Colo.), FACSAria^{™}, FACSArray^{™}, FACS Vantage^{™}, BD^{™} LSR II, and FACSCaiibur^{™} (BD Biosciences, San Jose, Calif.) and other equivalent cell sorters produced by other commercial vendors such as Sony, Bio-Rad, and Beckman Coulter.

In another embodiment, the invention relates to a method according to any one of the preceding embodiments as described herein, wherein reporter cells efficiently expressing MHC on their surface are enriched by MACS-based cell sorting.

"MACS" refers to a method of separating a population of cells into one or more sub-populations based on the presence, absence, or level of one or more MACS-selectable polypeptides expressed by the cells. MACS relies on magnetic susceptibility properties of tagged individual cells in order to sort the cells into sub-populations. For MACS, magnetic beads (such as those available from Miltenyi Biotec Bergisch Gladbach, Germany; 130-048-402) can be used as labels. MACS cell sorters suitable for carrying out a method described herein are well-known in the art and commercially available. Exemplary MACS cell sorters include autoMACS Pro Separator (Miltenyi Biotec).

In various embodiments, cells are contacted with an antibody specific for MHC-I or MHC-II or with antibodies detecting proteins associated with hybrids. For example, antibodies detecting the CD3γ, CD3δ or CD3ε can be used, if the MHC extracellular domains are fused to the TCR transmembrane regions. The antibody may be directly conjugated to a detectable label. Alternatively, a secondary antibody, conjugated to a detectable label and specific for the first antibody, may be contacted with the cells. Detectable labels suitable for use include any compound detectable by spectroscopic, photochemical, biochemical, immunochemical, electrical, optical or chemical means. Useful labels in the present invention include biotin, magnetic beads (e.g., Dynabeads^{™}), fluorescent labels (e.g., fluorescein, texas red, rhodamine, green fluorescent protein, dansyl, umbelliferone, PE, APC, CY5, Cy7, PerCP, Alexa dyes and the like), radiolabels (e.g., ³H, ¹²⁵1, ³⁵S, ¹ C, or ³²P), enzymes (e.g., horse radish peroxidase, alkaline phosphatase and others), and colorimefric labels such as colloidal gold or colored glass or plastic (e.g., polystyrene, polypropylene, latex, etc.) beads. A variety of suitable fluorescent labels are further described in, for example, The Molecular Probes Handbook: A Guide to Fluorescent Probes and Labeling Technologies, 11th Edition.

The sorting results in a population of non-fluorescent cells and at least one population of fluorescent cells, depending on how many fluorescent labels were used. The presence of at least one cell population with fluorescent cells is indicative that at least one candidate peptide is efficiently presented by APCs. Thus, FACS enables sorting of the population of cells to produce a population of cells enriched in cells that comprise surface-exposed MHC-I or MHC-II.

Embodiments of the present invention utilize methods of DNA isolation known to those skilled in the art. In general, the aim is to separate DNA present in the nucleus of the cell from other cellular components. The isolation of DNA usually begins with lysis or breakdown of cells. This process is essential for the destruction of protein structures and allows for release of nucleic acids from the nucleus. Lysis is carried out in a salt solution, containing detergents to denature proteins or proteases (enzymes digesting proteins), such as Proteinase K, or in some cases both. It results in the breakdown of cells and dissolving of membranes. Methods of DNA isolation include, but are not limited to, phenol:chloroform extraction, high salt precipitation, alkaline denaturation, ion exchange column chromatography, resin binding, and paramagnetic bead binding.

Embodiments of the present invention utilize methods of cDNA generation known to those skilled in the art. In general, the aim is to convert the isolated RNA present in the cells to DNA, co called copy-DNA, in order to use it as template for polymerase chain reaction (PCR). The isolation of RNA usually begins with lysis or breakdown of cells. This process is essential for the destruction of protein structures and allows for release of nucleic acids from it. Lysis is usually carried out in Phenol containing solution (e.g. TRIzol^{™}). It results in the breakdown of cells and dissolving of membranes and allows the separation of RNA from other cellular components. The isolated RNA is then converted into cDNA by reverse transcriptase (e.g. Superscript^{™}, Goscript^{™})

The sequence of the candidate peptides within the MHC-peptide complex presented on the cell surface is then amplified by PCR and may be sequenced by any method known in the art.

In various embodiments, the invention relates to a method according to any one of the preceding embodiments as described herein, wherein the sequence of the candidate peptides is determined by PCR and sequencing.

In one embodiment the sequence of the candidate peptides is determined by digital PCR. Digital polymerase chain reaction (digital PCR, DigitalPCR, dPCR, or dePCR) is a refinement of conventional polymerase chain reaction methods that can be used to directly quantify and clonally amplify nucleic acids including DNA, cDNA or RNA.

Sequencing may also be performed using microfluidics. Microfluidics involves micro-scale devices that handle small volumes of fluids. Because microfluidics may accurately and reproducibly control and dispense small fluid volumes, in particular volumes less than 1 µl, application of microfluidics provides significant cost-savings. The use of microfluidics technology reduces cycle times, shortens time-to-results, and increases throughput. Furthermore, incorporation of microfluidics technology enhances system integration and automation. Microfluidic reactions are generally conducted in microdroplets.

In some embodiments, sequencing is performed using Second Generation Sequencing (or Next Generation or Next-Gen), Third Generation (or Next-Next-Gen), or Fourth Generation (or N3-Gen) sequencing technology including, but not limited to, pyrosequencing, sequencing-by-ligation, single molecule sequencing, sequence-by-synthesis (SBS), massive parallel clonal, massive parallel single molecule SBS, massive parallel single molecule real-time, massive parallel single molecule real-time nanopore technology. Morozova and Marra provide a review of some such technologies in Genomics, 92: 255 (2008).

In some embodiments, prior to, following or concurrently with sequencing, nucleic acids are amplified. Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (e.g., PCR) require that RNA be reverse transcribed to DNA prior to amplification (e.g., RT-PCR), whereas other amplification techniques directly amplify RNA (e.g., TMA and NASBA).

In one embodiment, the invention relates to a method according to any one of the preceding embodiments as described herein, wherein the candidate peptide is for use as a vaccine.

In a particular embodiment, the vaccine is a tumor specific antigen (TSA)-based cancer vaccine.

The term "vaccination" or equivalents are well-understood in the art. For example, the term vaccination can be understood to be a process that increases a subject's immune reaction to antigen and therefore the ability to resist or overcome a disease. A "vaccine" is to be understood as meaning a composition for generating immunity for the prophylaxis and/or treatment of diseases (e.g. cancer). Accordingly, vaccines are medicaments which comprise antigens and are intended to be used in humans or animals for generating specific defense and protective substance by vaccination. The term "TSA-based cancer vaccine" is meant to refer to a vaccine containing a pooled sample of tumor-specific antigens, for example at least one, at least two, at least three, at least four, at least five, or more tumor-specific peptides. For a vaccine to be effective, a short linear peptide containing an antigen or epitope must be presented by an MHC at the surface of an APC. The set of MHCs variants expressed differs strongly between different individuals.

Recurrent tumor-derived mutations may serve as public tumor-specific antigens enabling the development of TSA-based cancer vaccines applicable to broader patient cohorts. Accordingly, the method of the present invention can be used for identifying patients containing certain MHC variants and efficiently presenting these common/public TSAs to the immune system. However, many tumor-derived mutations appear to derive from patient-specific alterations and any of those alterations may affect the binding of the TSA peptide to MHCs. Thus, the method of the present invention can also be used for identifying patient-specific candidate peptides for personalized vaccines.

Furthermore, the tumor of a patient often comprises many TSAs, whereof each may only be contained in a subset of tumor cells. In some embodiments, the set of candidate TSAs may therefore consist of TSAs derived from different sets of tumor cells. An important step in the development of a cancer vaccine is therefore to select from the TSAs candidates which are contained and preferably well expressed in the tumor cells of a patient, those with high affinity to the MHC expressed in that patient. For an optimal immune response, the set of TSAs comprised in a vaccine should further activate both CD4+ and CD8+ T-cells, which recognize MHCII or MHCI-peptide complexes, respectively. Thus, the method of the present invention can be used for identifying TSAs binding to specific MHCII and MHCI variants of a patient.

In one embodiment, the candidate peptide is for use to induce immunological tolerance against at least one of the epitopes it comprises. As used herein, "immunological tolerance" refers to a reduction in immunological reactivity of a host towards a specific antigen or antigens. The antigens comprise immune determinants/epitopes that, in the absence of tolerance, cause an unwanted immune response. Immunological tolerance can be induced to prevent or ameliorate transplant rejection, autoimmunity, allergic reaction, or another undesirable immune response. Without being bound by theory, immunological tolerance may be achieved by the generation of regulatory T cells which act as negative regulators of the immune response. The balance of different types of T-cells may also depend on the interaction of TCRs and MHC-peptide complexes. The method of the present invention may therefore be used to select MHC-peptide complexes suitable to generate immunological tolerance against that peptide. Other possible mechanisms to achieve immunological tolerance comprise the blocking of TCRs of specific T-cells and/or killing of specific T-cells.

Thus, in one embodiment, the candidate peptide is for use to block TCRs in the context of an MHC molecule. "Blocking of TCRs" refers to any agent which includes a peptide-MHC complex which blocks natural TCR-MHC interaction. To predict the TCR-blocking efficacy of a MHC-peptide complex, the stability of that complex is important.

In another embodiment, the candidate peptide is for use for MHC-mediated toxin delivery to cells, in particular to T-cells.

"MHC-mediated toxin delivery" refers to methods covalently linking toxic agents (proteins or other) to peptide-MHC tetramers or other MHC multimers in order to deliver the said toxin into the cell, in particular a T-cell, to cause the death of the cell. The method disclosed herein allows to measure the stability of specific MHC-peptide pairs and may thus be used to develop MHC-peptide complexes for the use of blocking specific TCRs or killing specific T-cells, in particular for *in vivo* applications.

In yet another embodiment, the candidate peptide is for use for redirecting T cells towards other peptide-MHC specific T cells via MHC-CARs.

Redirecting T cells towards other cells with CARs composed of antibodies linked to the intracellular chain of the CD247(CD3zeta) have been used commonly and are already in the clinic. T cells, in particular cytotoxic T cells, equipped with CARs composed of peptide-MHC linked to the intracellular chain of the CD247(CD3zeta) or T cells equipped with MCRs can use these receptors to recognize T cells specific for these peptide-MHC complexes and kill them (Jyothi et al., Nat.Biotech, 20, 1215-1220 (2002)). The method disclosed herein allows to measure the stability of specific MHC-peptide pairs and may thus be used to develop MHC-peptide complexes for the use in CARs or MCRs for redirected killing of specific T-cells, in particular for *in vivo* applications.

For many therapies modulating an immune response, the identification of TCRs involved in that immune response, is required. There are different ways, well known in the art, which allow identifying those TCRs and relying on the interaction of TCRs with an epitope contained in a MHC-peptide complex. A critical prerequisite is the information how well a MHC-peptide complex is presented at the surface of an APC. If an epitope is not presented by the MHCs of an APC used in an experimental in *vitro* assay, a suitable TCR binding to that epitope may not be identified. Furthermore, if in a library or MHC-peptide complexes presented by APCs in such an assay, one of the peptides is presented preferentially, this may lead to the selection of a suboptimal TCR. For an optimized TCR identification and selection process it is therefore important to overcome the uncertainty associated with the presentation of a MHC-peptide complex. The method of the present invention may therefore be used to provide quantitative information about the affinity of a specific peptide to a specific MHC and select suitable complexes for *in vitro* assays used for identifying TCRs recognizing an epitope or for enriching T-cells expressing such a TCR.

In one embodiment, the candidate peptide is for use for a T-cell reactivity test.

The term "T-cell reactivity" as used herein refers to the capability of a substance to elicit T-cell activation. More specifically, "T-cell reactivity" means the capability of a peptide to induce proliferation, differentiation and/or cytokine production of T cells. T-cell reactivity assays are well known in the art and often comprise co-culture of APCs presenting MHC-peptide complexes and T-cells. Those assays may be used to expand T-cells for cell therapies or to identify TCRs binding to a specific epitope, for example for adaptive T-cell therapies or chimeric antigen receptor (CAR) T-cell therapies. T-cell reactivity tests may be further used for immunogenicity testing.

In a certain embodiment, the candidate peptide is for use for immunogenicity testing. The term "immunogenicity testing" as used herein refers to measuring the potential immune responses to biotherapeutics. Biotherapeutics can elicit an immune response that may impact their safety and efficacy. Immunogenicity testing is employed to monitor and evaluate humoral (antibody) or cellular (T cells) responses during clinical and pre-clinical studies. Usually testing immunogenicity of a biotherapeutics involves measuring antibodies specifically generated against the biotherapeutics. With the method of the present invention it is possible to identify peptides that are efficiently presented by MHC molecules, in particular MHCs specific to an individual, and thus potentially elicit a T-cell mediated immune response, in an *in vitro* procedure. This can help to provide a more complete and accurate picture of the overall immunogenic profile of a compound, and at the same time may reduce burden of patients or clinical trial participants

In a further embodiment, the invention provides a MHC-peptide complex comprising a candidate peptide covalently bound to an extracellular part of the alpha or beta domain of the MHC molecule, an extracellular part of the MHC beta domain, and at least one transmembrane domain of a heterologous molecule.

In one embodiment, the invention provides a MHC-peptide complex according to the preceding embodiment, wherein the transmembrane domain is a transmembrane domain of a heterologous molecule such as TCRα/β, TCRγ/δ, CD3γ/δ/ε/ζ, CD4 or CD8α/β.

In one embodiment, the MHC molecule is a MHC class II molecule comprising the extracellular MHC class II alpha chain, the extracellular MHC class II beta chain, and at least two transmembrane domains.

In another embodiment, the MHC molecule is a MHC class I molecule, comprising the extracellular MHC class I alpha chain, the beta-2 macroglobulin, and at least one transmembrane domain.

In yet another embodiment, the MHC molecule is a MHC hybrid molecule comprising the fusion of peptide, β2-microglobulin and the extracellular MHC class I alpha chain and at least one transmembrane domain.

In a specific embodiment, the MHC alpha chain of the MHC class I molecule carries the Y84A mutation.

In various embodiments, the invention provides a MHC-peptide complex, wherein the candidate peptide is a tumor-specific peptide carrying individual tumor-derived mutation(s).

In a specific embodiment, the tumor-derived mutation is a single nucleotide variant (SNV).

In a further embodiment, the invention provides a recombinant construct comprising a nucleotide sequence coding for a MHC-peptide complex as defined in any one of the preceding embodiments.

In yet a further embodiment, the invention provides an expression cassette comprising a promoter sequence linked to the recombinant construct of the preceding embodiment.

In another embodiment, the invention provides a vector comprising the expression cassette of the preceding embodiment.

In yet another embodiment, the invention provides a cell comprising the MHC-peptide complex of the invention.

In one embodiment, the invention relates to the cell according to the preceding embodiment comprising a MHC class II peptide complex, wherein the cell is a mammalian cell lacking the MHC class II peptide loading machinery.

In another embodiment, the cell comprises a MHC class I peptide complex, wherein the cell is a mammalian cell lacking a functional TAP1, TAP2 and/or beta-2-microglobulin gene.

In yet another embodiment, the cell comprises a MHC class I peptide complex, wherein the cell is a T-cell hybridoma with a defective or deleted TAP1, TAP2 and/or beta-2-microglobulin gene.

Further comprised by the invention is a method for determining the MHC binding affinity of candidate peptides comprising expressing in a reporter cell line a recombinant MHC-peptide complex comprising a covalently bound candidate peptide, and detecting reporter cells that present the MHC-peptide complex on the surface of the reporter cell, including measurement of the level of such presentation/expression. The reporter cell line is a mammalian cell line. In one embodiment, the mammalian cell line used shows altered characteristics with respect to a native cell line, e.g. it carries enzyme deletions, mutations and/or overexpression of enzymes and/or MHC molecules.

In one embodiment, the invention relates to a method according to the preceding embodiment comprising:
(i) generating libraries comprising candidate peptides cloned upstream of a recombinant MHC alpha or beta domain connected to a transmembrane region;
(ii) transducing such libraries, together with the corresponding MHC alpha or beta domain, into suitable reporter cell lines;
(iii) detecting cells that present one or more MHC-peptide complex(es) on their cell surface.
(iv) determining the level of such presentation/expression.

The methods of the present invention may also be applied to high throughput screening. High throughput screening (HTS) technology is commonly used to define the rapid processing of cells on a large scale. In certain embodiments, a plurality of screens may be run in parallel with different candidate peptide libraries. High throughput screening systems are commercially available and typically automate entire procedures, including all sample and reagent pipetting, liquid dispensing, timed incubations, and final readings of the microplate in detector(s) appropriate for the assay. These configurable systems provide high throughput and rapid start up as well as a high degree of flexibility and customization.

By the term "peptide" as used herein is meant at least two covalently attached amino acids. Generally, MHC class I peptides are 8 or 9 amino acids in length, but can vary to between 7 and 10 amino acids in length. MHC class II peptides can vary from 15 to 24 amino acids in length. Optionally, they can vary from 10 amino acids to 30 amino acids or more in length.

As used herein, binding or "binding affinity" of a peptide to an MHC or interaction of a peptide with an MHC refers to the peptide-MHC interaction which occurs in the peptide-binding groove or cleft. Such a binding or interaction may also occur naturally.

"Covalent binding", or "tethering" of a peptide to an MHC, as used herein, refers to a recombinant MHC-peptide complex wherein the covalent bond is generated by genetic engineering.

A MHC-peptide complex, as used herein, refers to a complex wherein a peptide is bound to a MHC. This binding can occur via the peptide-binding groove or cleft and/or by covalent binding of a peptide to an MHC.

In a recombinant MHC-peptide complex, as used herein, a candidate peptide is always covalently bound to an MHC, but the peptide may or may not efficiently bind to the cleft of the MHC.

The stability of a MHC-peptide complex refers to the stability of the peptide-MHC interaction occurring in the peptide-binding groove which correlates with the surface expression of said MHC-peptide complex. It does not refer to the stability of the covalent peptide-MHC bond.

The term "antigen" as used herein refers to all, or parts, of a peptide or protein, capable of eliciting an immune response against itself or portions thereof. This immune response may involve either antibody production, or the activation of specific immunologically competent cells, or both.

The term "peptide loading machinery" as used herein refers to all required components necessary for the loading of MHC molecules with peptides. For example the MHC class I peptide loading machinery is present in almost all cells and includes among others the TAP transporter, tapasin, and calreticulin. The MHC class II peptide loading machinery is constantly present only in APCs, but it may be induced in other cells like T cells.

As used herein, the term "transmembrane domain" is defined as a predominantly hydrophobic sequence of amino acids that is capable of spanning a cell membrane. The term "transmembrane region" is used herein as a synonym for "transmembrane domain".

The term "library" or equivalents as used herein means a plurality of molecules. In the case of MHC-peptide complexes, the library provides a sufficiently structurally diverse population of peptides to effect a probabilistically sufficient range of cellular responses to provide one or more cells exhibiting a desired response. In a preferred embodiment, at least 10, preferably at least 50, more preferably at least 200 and most preferably at least 1000 peptides are simultaneously analyzed in the method of the invention. Libraries can be designed to maximize library size and diversity.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed or not expressed at all. Recombinant nucleic acid, is originally formed *in vitro,* in general, by the manipulation of nucleic acid, e.g., using polymerases and endonucleases, in a form not normally found in nature. In this manner, operably linkage of different sequences is achieved. Thus, an isolated nucleic acid, in a linear form, or an expression vector formed *in vitro* by ligating DNA molecules that are not normally joined, are both considered recombinant for the purposes of this invention. It is understood that once a recombinant nucleic acid is made and reintroduced into a host cell or organism, it will replicate non-recombinantly, i.e., using the *in vivo* cellular machinery of the host cell rather than *in vitro* manipulations; however, such nucleic acids, once produced recombinantly, although subsequently replicated non-recombinantly, are still considered recombinant for the purposes of the invention. Similarly, a recombinant protein, such as the MHC-peptide complex of the invention, is a protein made using recombinant techniques, i.e., through the expression of a recombinant nucleic acid as depicted above.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid, e.g., a promoter from one source and a coding region from another source. Similarly, a heterologous protein will often refer to two or more subsequences that are not found in the same relationship to each other in nature (e.g., a fusion protein).

The term "cancer" as used herein is defined as disease characterized by the rapid and uncontrolled growth of aberrant cells. Cancer cells can spread locally or through the bloodstream and lymphatic system to other parts of the body, Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

Furthermore, in the claims the word "comprising" does not exclude other elements or steps, and the indefinite article "a", "an", and "the" include plural referents unless the context clearly dictates otherwise.

Unless otherwise defined, all technical and scientific terms used herein have the same meaning as commonly understood by one of ordinary skilled in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Specific embodiments of the present invention are additionally illustrated by the following examples.

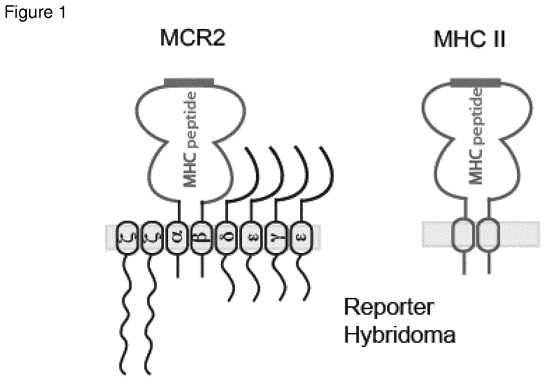

**Fig. 1** shows the structure of the MHC-TCR fusion molecule (MCR2) and the native MHC class II.

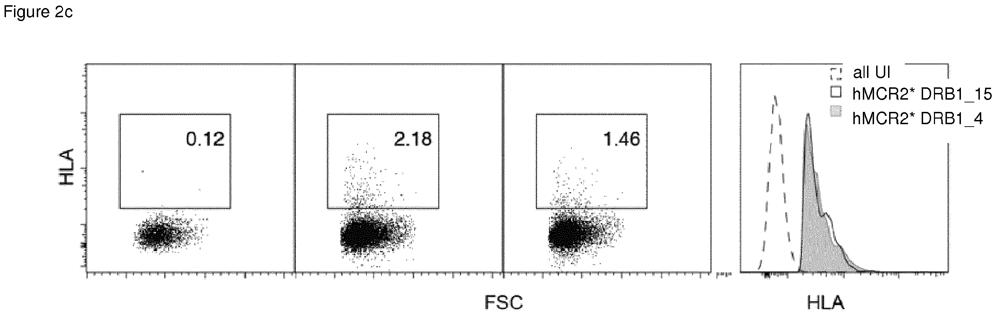

**Fig. 2** Constructs encoding MCRs carrying the alpha and beta chains of the human MHC molecules (HLA-DRB1_4, DRB1_15 and DRB5_1) with covalently linked influenza Matrix Protein 1 peptide MP1₁₀₃₋₁₂₀, (a), MP1₆₂₋₇₂ (b) or a library of influenza cDNA-derived peptides(c), have been made. After transduction of an MHC class II deficient cell line, anti-HLA-DR APC antibody was used to measure MCR surface expression by FACS. The graphs show efficient surface expression of the MCR containing HLA-DRB1_4-MP1₁₀₃₋₁₂₀ complex, while the DRB1_15-MP1₁₀₃₋₁₂₀ containing MCRs were detectable at very low levels (a). We barely detected MCRs containing the DRB5_1-MP1₁₀₃₋₁₂₀(a), but did detect efficient expression of DRB5_1-MP1₆₂₋₇₂ containing MCRs (b). Importantly, when a library of influenza cDNA-derived peptides was tethered to the HLA DBR 1_4 or HLA DRB 1_15, similar levels of MCR surface expression were observed (c). "UI" means uninfected cells.

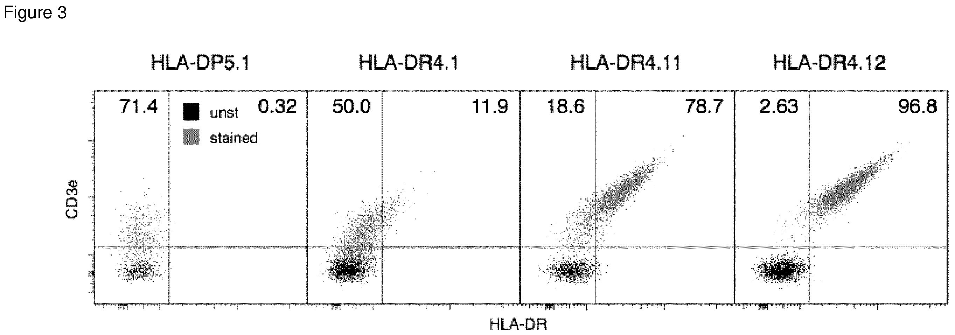

**Fig. 3** shows example analysis of cells transduced with MCRs containing various HLA beta chains carrying different peptides (pep1, pep2 and pep3), stained with anti-HLA-DR and anti-CD3e. Double-positive staining indicates that both antibodies are equally efficient in detecting MCR surface expression and the diagonal shape of the population, efficient association of the MCR with the CD3 chains.
**Fig. 4** shows low surface expression of MHC and MCR molecules without covalently linked peptides. Cells transduced with HLA-DRB1.4 (left) or MCR2-DRB1.4 constructs (right) were stained with either αHLA-DR (top) or αCD3 (bottom).

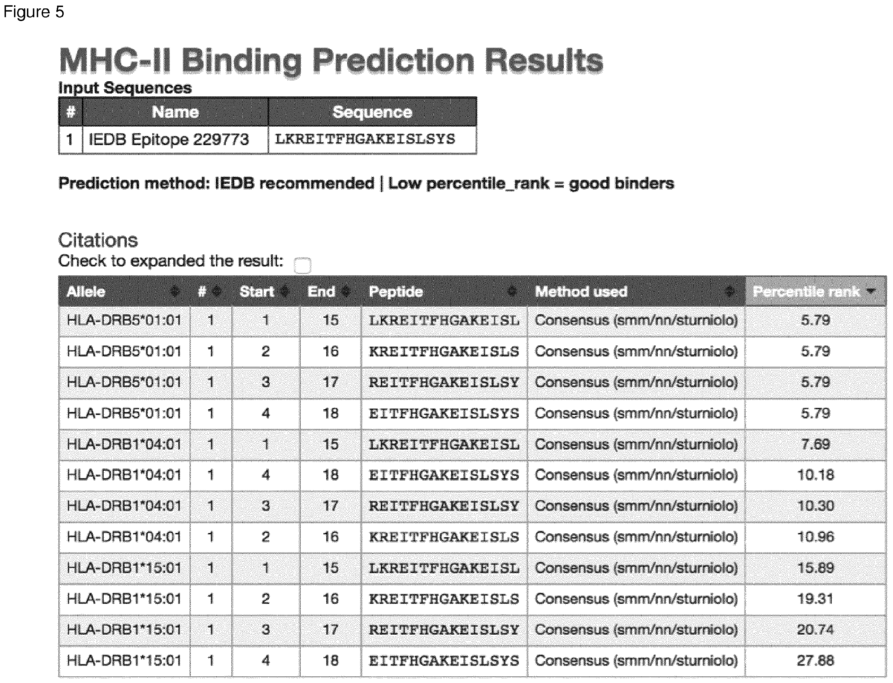

**Fig. 5** shows the peptide binding predictions for MP1₁₀₃₋₁₂₀ using the Immune Epitope Database (IEDB) analysis resource.

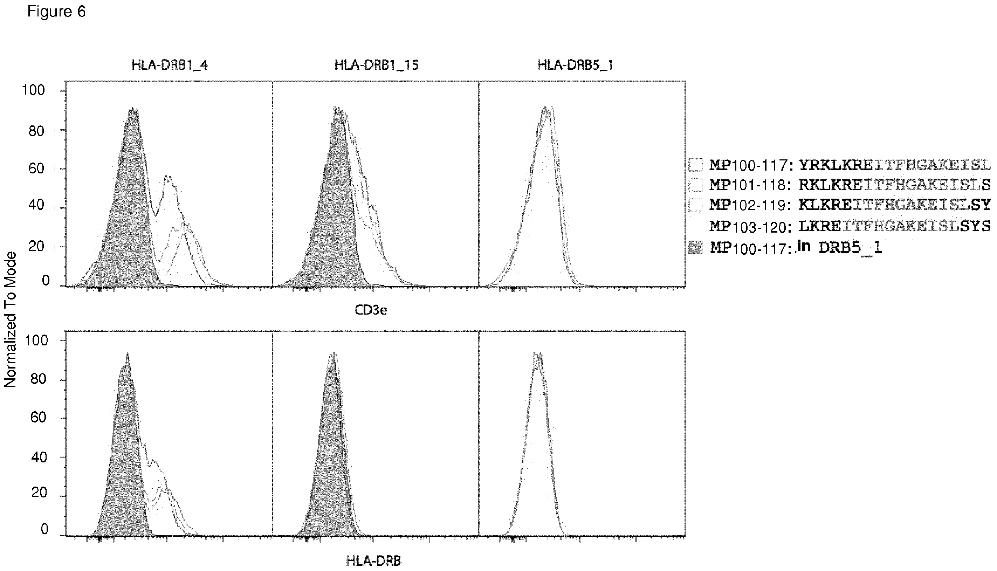

Fig. 6 shows FACS analysis of cells transduced with MCRs containing various HLA-DRB chains carrying different peptides containing the core epitope MP1₁₀₇₋₁₁₇. Anti-CD3e or anti-HLA-DR PE antibodies were used to measure MCR surface expression. The graphs show that the core MP1₁₀₇₋₁₁₇ peptide can efficiently be presented only on HLA-DRB1_4 and not by HLA-DRB1_15 nor HLA-DRB5_1 and that flanking peptide regions do not play a major role. The presence of the MCRs in all the samples was confirmed by PCR and sequencing (not shown).
Fig. 7. A library of random peptides tethered to the alpha chain of the mouse MCR was transduce into reporter cells, single cells expressing the MCR on the surface were sorted and the sequence of example clones was determined. The graph represents a FACS analysis of example clones carrying MCRs with such peptides, stained with anti-mouse MHC class II and the corresponding peptide sequences.

### Example 1 - Differential surface expression of MCRs containing influenza Matrix Protein 1 peptide

The inventors constructed MHC-TCR fusion proteins (MCRs) composed of peptides linked to native extracellular domains of the MHC fused to trans-membrane units of the TCR (Fig. 1; see also WO 2016/097334 A1). In particular, constructs encoding MCR carrying the alpha and beta chains of the human MHC molecules (HLA-DRB1_4, DRB1_15 and DRB5_1) with the beta chains carrying covalently linked influenza Matrix Protein 1 peptide (MP1₁₀₃₋₁₂₀), MP1₆₂₋₇₂ or a library of influenza cDNA-derived peptides, have been generated. Retrovirus containing supernatants were produced according to standard protocol in the ecotropic Phoenix packaging cell line and used to infect reporter cell lines and sorted cells. After transduction of an MHC class II deficient cell line (T cell hybridoma), anti-HLA-DR APC antibody were used to measure MCR surface expression by FACS (Fig. 2). The MP1-derived peptide comprising amino acids 103-120 tethered to the HLA DRB 1_4 leads to efficient expression of the MCR on the surface of the mammalian reporter cell line. The same peptide tethered to HLA DRB 1_15 leads to very low MCR expression and in the context of HLA DRB 5_1 is not expressed at all (Fig. 2a). However, another MP1-derived peptide comprising amino acids 62-72 leads to efficient expression of the MCR containing the HLA DRB 5-1, but not the other HLAs tested (Fig. 2b). Importantly, when a library of influenza cDNA-derived peptides was tethered to the HLA DBR 1_4 or HLA DRB 1_15, similar levels of surface expression were observed, indicating that both HLA 1_4 and HLA 1_15 can be efficiently expressed on cell surface as parts of the MCR (Fig. 2c). It is known from published studies (Schmid et al Immunity 2007) that the MP1 peptides comprising amino acids 103-117 and 107-121 are efficiently presented by the HLA DRB 1-4, suggesting that only if the covalently attached peptide efficiently binds to the MHC peptide-binding groove, the MHC complex is sufficiently stable for surface expression that can be detected by antibody staining. Figure 6 furthermore shows that that the core MP1₁₀₇₋₁₁₇ peptide can efficiently be presented only on HLA-DRB1_4 irrespectively to the flanking peptide regions.

### Example 2 - Detection of MCR surface expression with different antibodies

Surface expression of the MCRs containing various HLA beta chains carrying different peptides (pep1, pep2 and pep3), was detected with antibody staining using anti-HLA-DR and anti-CD3e and analyzed on BDFortessa flow cytometer (Fig.3). Double-positive staining indicates that both antibodies are efficient in detecting MCR surface expression and that anti-CD3e staining can be used universally to detect MCRs carrying different HLA alleles. The diagonal shape of the population further indicates efficient association of the MCR with the CD3 chains.

### Example 3 - MHC binding affinity predictions for MP1₁₀₃₋₁₂₀

The MHC binding affinity for MP1₁₀₃₋₁₂₀ was predicted using the Immune Epitope Database (IEDB) analysis resource (Wang, P. et al. A Systematic Assessment of MHC Class II Peptide Binding Predictions and Evaluation of a Consensus Approach. PLoS Comput Biol 4, e1000048 (2008); Wang, P. et al. Peptide binding predictions for HLA DR, DP and DQ molecules. BMC Bioinformatics 11, 568 (2010)). For MP1₁₀₃₋₁₂₁ the method of the present invention indicated HLA DRB 4_1 to bind best and lead to high surface expression of the HLA, HLA DRB 1_15 to bind moderately and lead to low surface expression and HLA DRB 5_1 not to bind at all. However, using the IEDB analysis resource, the peptide-binding prediction analysis indicated HLA DRB 5_1 to be the best MP1₁₀₃₋₁₂₀ binder (Fig.5).

### Example 4 - Detection of surface expression of MCRs containing peptides fused to the alpha chain.

The inventors constructed a library of mouse MCRs carrying random peptides tethered to the alpha chain. After transduction of an MHC class II deficient cell line (T cell hybridoma), anti-MHC antibody was used to measure MCR surface expression by FACS (Fig. 7). Efficient expression of MCRs carrying many different peptides was detected, indicating that peptides linked to the alpha chain of the MCR can also stabilize the MHC complex enough for surface expression.

## Claims

1. A method for identifying a candidate peptide to be presented by a major histocompatibility complex (MHC), the method comprising the steps of:
a. expressing a recombinant MHC-peptide complex in a reporter cell line, wherein the recombinant MHC-peptide complex is a peptide-MHC-TCR chimera in which the candidate peptide is covalently bound to the MHC and wherein the reporter cell line is a mammalian cell line;
b. detecting and isolating reporter cells that express one or more recombinant MHC-peptide complex(es) on the cell surface, wherein the detection and isolation of reporter cells comprises a step of contacting the reporter cells with an antibody directed against the recombinant MHC-peptide complex;
c. determining the amino acid sequence of the candidate peptide presented on the cell surface of a reporter cell isolated in step (b); and
d. identifying the candidate peptide of step (c) to be presented by an MHC.

2. The method according to claim 1, wherein the MHC is:
a. an MHC class II molecule comprising an extracellular MHC class II alpha chain and a transmembrane domain, and an extracellular MHC class II beta chain and a transmembrane domain; or
b. an MHC class I molecule comprising an extracellular MHC class I alpha chain and a transmembrane domain, and a beta-2 microglobulin.

3. The method according to claim 2, wherein the MHC class I alpha chain comprises a Y84A mutation.

4. The method according to claim 2 or 3, wherein the transmembrane domain is a native transmembrane domain of an MHC molecule or a transmembrane domain of a heterologous molecule, in particular wherein the heterologous molecule is TCRα/β, TCRγ/δ, CD3γ/δ/ε/ζ, CD4 or CD8α/β.

5. The method according to any one of claims 1 to 4, wherein the antibody is directed against MHC-I or MHC-II, or wherein the antibody is directed against CD3γ, CD3δ or CD3ε.

6. The method according to any one of claims 1 to 5, wherein reporter cells efficiently expressing a recombinant MHC-peptide complex on their cell surface are isolated by FACS or MACS.

7. The method according to any one of claims 1 to 6, wherein the reporter cell line is a cell line lacking the MHC class II peptide loading machinery and/or lacking a functional TAP1, TAP2 and/or beta-2-microglobulin gene.

8. The method according to any one of claims 1 to 7, wherein the reporter cell line is a T cell hybridoma cell line.

9. The method according to any one of claims 1 to 8, wherein the candidate peptide is encoded in an MHC-peptide complex library.

10. The method according to claim 9, wherein the MHC-peptide library is obtained by cloning a plurality of polynucleotides encoding the candidate peptides upstream of and in-frame with a recombinant MHC beta and/or alpha chain.

11. The method according to claim 10, wherein the plurality of polynucleotides encoding the candidate peptides is obtained:
a. by mutagenesis of one or more polynucleotides encoding native peptide(s); or
b. by random sheering or digestion of cDNA or DNA derived from cells or pathogens of interest.

12. The method according to any one of claims 9 to 11, wherein the MHC-peptide library encodes at least 10, at least 50, at least 200 or at least 1000 peptide candidates.

13. The method according to any one of claims 9 to 12, wherein the MHC-peptide library is encoded by a plurality of viral vectors.

14. The method according to any one of claims 1 to 13, the method comprising an additional step of determining the level of cell surface expression of the recombinant MHC-peptide complex.

15. The method according to any one of claims 1 to 14, wherein the candidate peptide is a tumor-specific peptide carrying individual tumor-derived mutation(s).

## Patentansprüche

1. Verfahren zum Identifizieren eines Kandidatenpeptids, das von einem Haupthistokompatibilitätskomplex (major histocompatibility complex; MHC) präsentiert wird, wobei das Verfahren die Schritte aufweist:
a. Exprimieren eines rekombinanten MHC-Peptid-Komplexes in einer Reporterzelllinie, wobei der rekombinante MHC-Peptid-Komplex eine Peptid-MHC-TCR-Chimäre ist, in der das Kandidatenpeptid kovalent an den MHC gebunden ist und wobei die Reporterzelllinie eine Säugerzelllinie ist;
b. Nachweisen und Isolieren von Reporterzellen, die einen oder mehrere rekombinante(n) MHC-Peptid-Komplex(e) auf der Zelloberfläche exprimieren, wobei der Nachweis und die Isolierung von Reporterzellen einen Schritt des Inkontaktbringens der Reporterzellen mit einem Antikörper, der gegen den rekombinanten MHC-Peptid-Komplex gerichtet ist, umfassen;
c. Bestimmen der Aminosäuresequenz des Kandidatenpeptids, das auf der Zelloberfläche einer in Schritt (b) isolierten Reporterzelle präsentiert wird; und
d. Identifizieren des Kandidatenpeptids aus Schritt (c), das von einem MHC präsentiert wird.

2. Verfahren nach Anspruch 1, wobei der MHC:
a. ein MHC-Klasse II-Molekül ist, das eine extrazelluläre MHC-Klasse II-alpha-Kette und eine Transmembrandomäne und eine extrazelluläre MHC-Klasse II-beta-Kette und eine Transmembrandomäne umfasst; oder
b. ein MHC-Klasse I-Molekül ist, das eine extrazelluläre MHC-Klasse I-alpha-Kette und eine Transmembrandomäne und ein beta-2-Mikroglobulin umfasst.

3. Verfahren nach Anspruch 2, wobei die MHC-Klasse I-alpha-Kette eine Y84A-Mutation umfasst.

4. Verfahren nach Anspruch 2 oder 3, wobei die Transmembrandomäne eine native Transmembrandomäne eines MHC-Moleküls oder eine Transmembrandomäne eines heterologen Moleküls ist, insbesondere wobei das heterologe Molekül TCRα/β, TCRγ/δ, CD3γ/δ/ε/ζ , CD4 oder CD8α/β ist.

5. Verfahren nach einem der Ansprüche 1 bis 4, wobei der Antikörper gegen MHC-I oder MHC-II gerichtet ist, oder wobei der Antikörper gegen CD3γ, CD3δ oder CD3ε gerichtet ist.

6. Verfahren nach einem der Ansprüche 1 bis 5, wobei die Reporterzellen, die wirksam einen rekombinanten MHC-Peptid-Komplex auf ihrer Zelloberfläche exprimieren, mit FACS oder MACS isoliert werden.

7. Verfahren nach einem der Ansprüche 1 bis 6, wobei die Reporterzelllinie eine Zelllinie ist, der die MHC-Klasse II-Peptid-Beladungsmaschinerie und/oder ein funktionelles TAP1-, TAP2- und/oder beta-2-Mikroglobulin-Gen fehlt.

8. Verfahren nach einem der Ansprüche 1 bis 7, wobei die Reporterzelllinie eine T-Zell-Hybridomzelllinie ist.

9. Verfahren nach einem der Ansprüche 1 bis 8, wobei das Kandidatenpeptid in einer MHC-Peptid-Komplexbibliothek codiert ist.

10. Verfahren nach Anspruch 9, wobei die MHC-Peptid-Bibliothek durch Clonieren einer Vielzahl von Polynucleotiden erhalten wird, die die Kandidatenpeptide stromaufwärts von und im Leserahmen (in-frame) mit einer rekombinanten MHC-beta- und/oder -alpha-Kette codieren.

11. Verfahren nach Anspruch 10, wobei die Vielzahl von Polynucleotiden, die die Kandidatenpeptide codieren, erhalten wird:
a. durch Mutagenese eines oder mehrerer Polynucleotide, die (ein) native(s) Peptid(e) codieren; oder
b. durch Zufallsscherung oder -verdau von cDNA oder DNA, die von Zellen oder Pathogenen von Interesse stammen.

12. Verfahren nach einem der Ansprüche 9 bis 11, wobei die MHC-Peptid-Bibliothek mindestens 10, mindestens 50, mindestens 200 oder mindestens 1000 Peptidkandidaten codiert.

13. Verfahren nach einem der Ansprüche 9 bis 12, wobei die MHC-Peptid-Bibliothek von einer Vielzahl von viralen Vektoren codiert wird.

14. Verfahren nach einem der Ansprüche 1 bis 13, wobei das Verfahren einen zusätzlichen Schritt des Bestimmens des Spiegels der Zelloberflächenexpression des rekombinanten MHC-Peptid-Komplexes umfasst.

15. Verfahren nach einem der Ansprüche 1 bis 14, wobei das Kandidatenpeptid ein Tumorspezifisches Peptid ist, das (eine) einzelne von einem Tumor abgeleitete Mutation(en) trägt.

## Revendications

1. Procédé d'identification d'un peptide candidat devant être présenté par un complexe majeur d'histocompatibilité (MHC), le procédé comprenant les étapes de :
a. expression d'un complexe MHC-peptide recombinant dans une lignée cellulaire rapporteuse, dans laquelle le complexe MHC-peptide recombinant est une chimère peptide-MHC-TCR dans laquelle le peptide candidat est lié de manière covalente au MHC et dans laquelle la lignée cellulaire rapporteuse est une lignée cellulaire de mammifère ;
b. détection et isolement de cellules rapporteurs qui expriment un ou plusieurs complexes MHC-peptide recombinants sur la surface cellulaire, la détection et l'isolement des cellules rapporteuses comprenant une étape de mise en contact des cellules rapporteuses avec un anticorps dirigé contre le complexe MHC-peptide recombinant ;
c. détermination de la séquence d'acides aminés du peptide candidat présenté sur la surface cellulaire d'une cellule rapporteuse isolée à l'étape (b) ; et
d. identification du peptide candidat de l'étape (c) devant être présenté par un MHC.

2. Procédé selon la revendication 1, dans lequel le MHC est :
a. une molécule du MHC de classe II comprenant une chaîne alpha extracellulaire du MHC de classe II et un domaine transmembranaire, et une chaîne bêta extracellulaire du MHC de classe II et un domaine transmembranaire ; ou
b. une molécule du MHC de classe I comprenant une chaîne alpha extracellulaire du MHC de classe I et un domaine transmembranaire, et une microglobuline bêta-2.

3. Procédé selon la revendication 2, dans lequel la chaîne alpha du MHC de classe I comprend une mutation Y84A.

4. Procédé selon la revendication 2 ou 3, dans lequel le domaine transmembranaire est un domaine transmembranaire natif d'une molécule de MHC ou un domaine transmembranaire d'une molécule hétérologue, en particulier dans lequel la molécule hétérologue est TCRα/β, TCRγ/δ, CD3γ/δ/ε/ζ, CD4 ou CD8α/β.

5. Procédé selon l'une quelconque des revendications 1 à 4, dans lequel l'anticorps est dirigé contre MHC-I ou MHC-II, ou dans lequel l'anticorps est dirigé contre CD3γ, CD3δ ou CD3ε.

6. Procédé selon l'une quelconque des revendications 1 à 5, dans lequel des cellules rapporteuses exprimant efficacement un complexe MHC-peptide recombinant sur leur surface cellulaire sont isolées par FACS ou MACS.

7. Procédé selon l'une quelconque des revendications 1 à 6, dans lequel la lignée cellulaire rapporteuse est une lignée cellulaire dépourvue de la machinerie de chargement peptidique de classe II du MHC et/ou dépourvue d'un gène fonctionnel TAP1, TAP2 et/ou microglobuline bêta-2.

8. Procédé selon l'une quelconque des revendications 1 à 7, dans lequel la lignée cellulaire rapporteuse est une lignée cellulaire d'hybridome de cellule T.

9. Procédé selon l'une quelconque des revendications 1 à 8, dans lequel le peptide candidat est codé dans une bibliothèque de complexes MHC-peptide.

10. Procédé selon la revendication 9, dans lequel la bibliothèque MHC-peptide est obtenue par clonage d'une pluralité de polynucléotides codant pour les peptides candidats en amont d'une chaîne bêta et/ou alpha MHC recombinante et en cadre avec celle-ci.

11. Procédé selon la revendication 10, dans lequel la pluralité de polynucléotides codant pour les peptides candidats est obtenue :
a. par mutagenèse d'un ou plusieurs polynucléotides codant pour un ou plusieurs peptides natifs ; ou
b. par cisaillement ou digestion aléatoire d'ADNc ou d'ADN dérivé de cellules ou d'agents pathogènes d'intérêt.

12. Procédé selon l'une quelconque des revendications 9 à 11, dans lequel la bibliothèque MHC-peptide code pour au moins 10, au moins 50, au moins 200 ou au moins 1 000 peptides candidats.

13. Procédé selon l'une quelconque des revendications 9 à 12, dans lequel la bibliothèque MHC-peptide est codée par une pluralité de vecteurs viraux.

14. Procédé selon l'une quelconque des revendications 1 à 13, le procédé comprenant une étape supplémentaire de détermination du niveau d'expression de surface cellulaire du complexe MHC-peptide recombinant.

15. Procédé selon l'une quelconque des revendications 1 à 14, dans lequel le peptide candidat est un peptide spécifique d'une tumeur portant une ou plusieurs mutations individuelles dérivées d'une tumeur.
